# EUROPEAN PATENT APPLICATION

(11) **EP 4 197 526 A1**
(43) Date of publication of application: **21.06.2023**
(21) Application number: 22214353.9
(22) Date of filing: 16.12.2022
(51) Int. Cl.: A61K 9/00, A61K 9/06

(54) **HYDROGEL FORMULATION COMPRISING ZEBULARINE AND RETIONIC ACID**

(30) Priority: 20.12.2021 PL 43991221
(71) Applicant: Uniwersytet Gdanski, 80-309 Gdansk (PL); Politechnika Gdanska, 80-233 Gdansk (PL); Gdanski Uniwersytet Medyczny, 80-210 Gdansk (PL); Innovabion sp.z o.o., 80-287 Gdansk (PL)
(72) Inventor: Skowron, Piotr, 80-303 Gdansk (PL); Rodziewicz-Motowidlo, Sylwia, 83-010 Rotmanka (PL); Dzierzynska, Maria, 81-824 Sopot (PL); Sawicka, Justyna, 80-174 Gdansk (PL); Sachadyn, Pawel, 81-464 Gdynia (PL); Slonimska, Paulina, 80-746 Gdansk (PL); Sosnowski, Pawel, 19-200 Ruda (PL); Sass, Piotr, 81-577 Gdynia (PL); Kaminska, Jolanta, 16-300 Augustow (PL); Baczynski-Keller, Jakub, 81-107 Gdynia (PL); Platek, Rafal, 02-972 Warszawa (PL); Deptula, Milena, 80-174 Gdansk (PL); Pikula, Michal, 80-170 Gdansk (PL)
(74) Representative: Pawlowska, Justyna

(57) **Abstract**

A pharmaceutical composition comprising two hydrogel formulations with biologically active substances for simultaneous use, one mixture of which comprises not less than 60 and not more than 240 mg of zebularine added to 1 ml of sodium alginate hydrogel, and the other mixture of which comprises not less than 1 and not more than 4 mg of retinoic acid added to 1 ml of sodium alginate hydrogel, wherein the biologically active substances, i.e. zebularine and retinoic acid, release gradually from each formulation due to hydrogel biodegradation, which allows their combined action to be used to stimulate complex tissue regeneration.

## Description

The invention refers to the simultaneous use of two alginate-based formulations containing zebularine and retinoic acid - composition of two formulations each constituting an active ingredient, which when administrated to the body biodegrades and gradually releases an active substance, zebularine and retinoic acid, which in turn promote tissue regeneration. The invention is used as a formulation to stimulate tissue regeneration processes, especially skin, in cases where topical administration of a regenerative drug is ineffective as it does not ensure adequate penetration of said drug to tissue and in case of long-term treatment damage. Examples of such lesions are chronic wounds and peripheral neuropathies.

Tissue regeneration is the process of restoring damaged or lost tissues leading to the recovery of the original structure and function. Regeneration processes may take place in the body spontaneously or as a result of therapy. The organisms of humans and other mammals depend on physiological regeneration necessary for normal functioning, as in the case of regular renewal of the epidermis, intestinal epithelium, endometrial epithelium or liver tissues. Tissue damage by external factors resulting from mechanical trauma, burns, toxins, radiation, ischemia or surgical operations can be repaired in the natural healing process. However, the effectiveness of endogenous regenerative mechanisms is not always sufficient to thoroughly rebuild the lost structures, or the progress of repair processes is too slow and is not completed in the time expected for a given type of damage. For example, the skin possesses solid regenerative abilities, but due to diabetic complications, such as ischemia, chemotherapy, and bedsores, skin wound healing may be significantly delayed, i.e. exceeding three months. In more severe cases, non-healing wounds may even become chronic. Non-healing wounds need therapeutic interventions. One of the said options is the pharmacological activation of the repressed regenerative potential. A promising but relatively understudied approach is the use of epigenetic inhibitors in combination with transcription activators. The combined administration of the DNA methyltransferase inhibitor - zebularine and the transcription activator - retinoic acid was proved to induce regeneration of ear pinna punch wounds in mice. Of note, ear punch wounds in laboratory mice that do not close spontaneously (Sass, P., et al. 2019, EBioMedicine 46: 317-329). However, the repair process can take a long time, which requires an extended treatment with regeneration-stimulating drugs. Repeated administration of the drug, e.g. in the form of injections, is troublesome, but it is also associated with rapid changes in the drug level, poorly tolerated by the organism and potentially disturbing the regeneration process. The so-far-developed scheme for administering zebularine and retinoic acid has been demonstrated in the patent application EP18000264.4A. A pharmaceutical composition is described as comprising zebularine and/or a pharmaceutically acceptable salt thereof as well as retinoic acid and/or retinoic acid metabolites and/or pharmaceutically acceptable salts thereof, and at least one known pharmaceutically acceptable carrier and/or diluent for use as an agent to promote regeneration or healing wounds caused by mechanical, chemical, thermal damage, surgical operations or pathological conditions. Treatment with this composition required 7 intraperitoneal injections of zebularine in 10 days combined with 6 intraperitoneal injections of retinoic acid in 11 days. Such a schedule is inconvenient to execute and does not provide a gradual release of the active ingredient.

Hence the need to develop more effective methods of tissue regeneration.

The subject of the invention is the simultaneous use of two hydrogel formulations in the composition as fololows: mixtures - formulation - based on alginate hydrogel comprising zebularine - the biologically active ingredient, and sodium alginate constituting the first formulation of the composition; retinoic acid - the biologically active ingredient and sodium alginate constituting the second formulation of the composition, which when administered subcutaneously into the body biodegrade and gradually release their active substances, i.e. zebularine and retinoic acid, which activate tissue regeneration. The term "simultaneous use" is understood to mean direct administration for this medical application - tissue regeneration to use both biologically active ingredients simultaneously, e.g. in two subcutaneous injections or a form administered directly to the lesion site but administered consecutively without delay. The invention, therefore, relates to a pharmaceutical composition - a set - comprising both formulations. The first formulation comprises sodium alginate and zebularine, and the second comrises retinoic acid and sodium alginate. The experiments established the proportions of zebularine substances and retinoic acid to alginate in each formulation. In the first formulation, zebularine is in a proportion corresponding to 60 to 240 mg per 1 ml of alginate hydrogel comprising 10 to 20 mg of sodium alginate alone in 1 ml of water, i.e. 1-2% sodium alginate. In the second formulation, retinoic acid is in the proportion of 1 to 4 mg per 1 ml of alginate hydrogel - comprising 10 to 20 mg of sodium alginate alone in 1 ml of water, i.e. 1-2% sodium alginate solution. Preferably, the formulation with retinoic acid additionally has an oil, e.g. rapeseed oil.

The invention is based on the simultaneous use of an alginate-based hydrogel formulation of zebularine and retinoic acid, delivered, e.g. in two subcutaneous injections or administered directly to the lesion site. Hydrogel formulations of zebularine and retinoic acid introduced under the skin gradually release active substances while subject to biodegradation. The gradually released active substances, zebularine and retinoic acid, together stimulate the regeneration of tissues at the lesion site that may be distant from the injection site.

The invention enables the administration of drugs in the form of biodegradable and biocompatible hydrogel compositions, whereby the drug is released gradually after a single administration, even over many days.

A more detailed description of the formulation composition is given below.

Hydrogel formulations were prepared as follows. The zebularine formulation was prepared by suspending from 60 to 240 mg of zebularine per 1 ml of alginate hydrogel. The retinoic acid formulation was prepared by suspending from 1 to 4 mg of retinoic acid per 1 ml of alginate hydrogel. The formulation comprising up to 240 mg of zebularine in 1 ml of 1-2% sodium alginate hydrogel is highly viscous but remains fluid and is suitable for subcutaneous injections or direct administration to the lesion site. The developed range from 60 to 240 mg shows effectiveness. The formulation comprising up to 4 mg of retinoic acid in 1 ml of 1-2% sodium alginate hydrogel is viscous yet retains a fluid form suitable for subcutaneous injection or direct administration to the lesion site. The developed range from 1 to 4 mg shows effectiveness. The contents of zebularine and retinoic acid in the prepared formulations were determined taking into account biologically active doses and based on the experiments testing the amounts of active substances that can be added to the hydrogel formulation while retaining the rheological properties suitable for injections or direct administration to the lesion site.

The formulation of zebularine and retinoic acid allowed a single subcutaneous administration in laboratory mice of a maximum of 48 mg of zebularine in 0.2 ml of hydrogel (240 mg per 1 ml) and a maximum of 0.8 mg of retinoic acid in 0.2 ml of hydrogel (4 mg per 1 ml), which corresponds to doses of approximately 2000 mg/kg and 32 mg/kg body weight, respectively. A mouse ear injury model was used to study the regenerative response. This model allows the observation of complex tissue regeneration involving the skin, cartilage, muscles, blood vessels, peripheral nerves and hair follicles. Hydrogel formulations of 2% sodium alginate with zebularine and retinoic acid administered subcutaneously in mice allowed mouse ear pinna regeneration. A detailed description of the experiment is shown in Example 1. Since the active substances administered in the hydrogel are gradually released under the skin, they are well tolerated by the body despite the high dose. Due to their consistency, the hydrogels with the active substance can also be applied directly to the lesion.

The invention is shown in more detail in the examples and in the drawings, which:

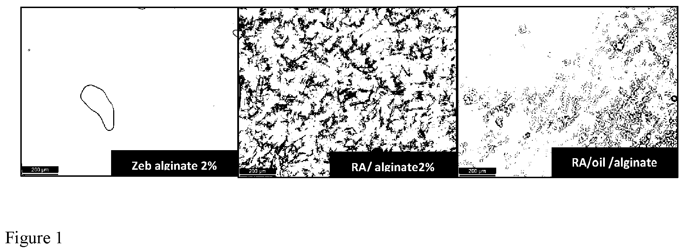

Figure 1 shows microscopic pictures of 2% alginate hydrogel formulations with 240 mg of zebularine (Zeb) per 1 ml alginate hydrogel - aqueous sodium alginate solution, 4 mg of retinoic acid (RA) per 1.0 ml of alginate hydrogel or 4 mg of retinoic acid in 0.1 ml of rapeseed oil per 1 ml of alginate hydrogel. Arrows indicate crystals of retinoic acid.
   The first formulation contains 240 mg of zebularine and 20 mg of sodium alginate in 1 ml water. The second mixture contains 4 mg of retinoic acid and 20 mg of sodium alginate in 1 ml water. The contents of zebularine and retinoic acid significantly exceed those corresponding to the solubility of these chemical compounds in water, which, according to the literature data, are up to 50 mg/ml for zebularine (Sass, P., et al. 2019, EBioMedicine 46: 317-329) and 0.000063 mg/ml (0.21 microM) for retinoic acid (Szuts EZ and Harosi FI 1991 Archives of Biochemistry and Biophysics287: 297-304). Concluding, the alginate hydrogel enables the effective administration of many times higher doses of zebularine and retinoic acid than aqueous solutions of the same volume.

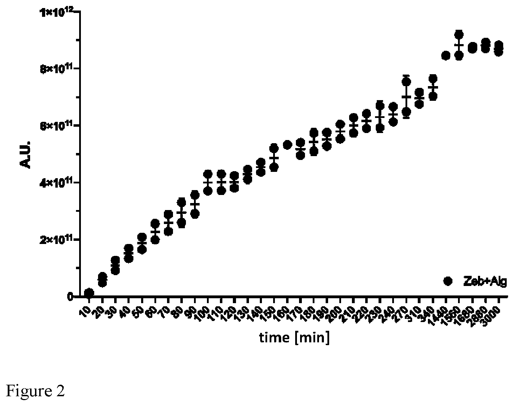

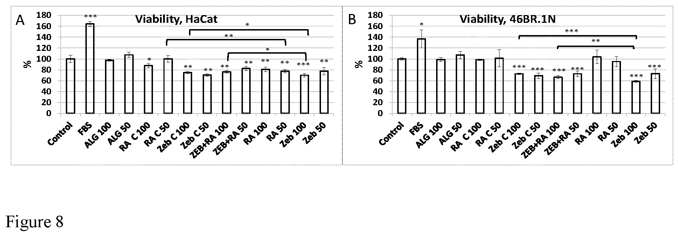

Figure 2 shows the result of an *in vitro* study of zebularine (Zeb) release from alginate hydrogel in a Franz chamber. No release of retinoic acid was detected in a Franz chamber, i.e. *in vitro. In vivo,* it is released under the skin due to alginate biodegradation.

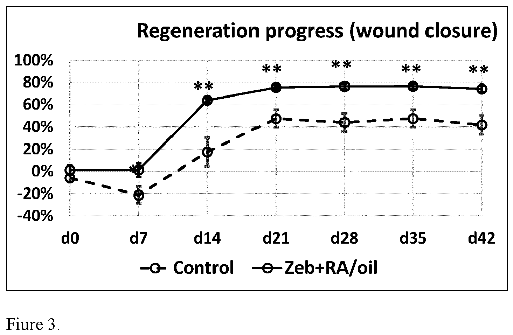

Figure 3 shows the progress of ear hole closure determined immediately after the injujry and on days 7, 14, 21, 28, 35 and 42 post-injury in 6 mice after administration of a 2% alginate hydrogel formulation with zebularine (Zeb) and retinoic acid (RA) suspended in rapeseed oil (n = 12 ears) and 6 control mice (n = 11 ears). The statistical significance of the results was determined using the Mann-Whitney two-tailed test. Differences with statistical significance p <0.05 are marked with an asterisk (*), p <0.01 with two asterisks (**).

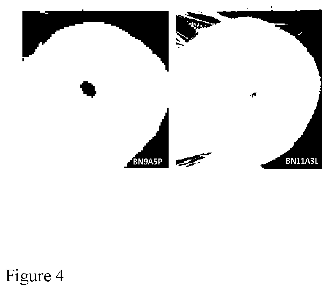

Figure 4 shows a representative photograph of the ear pinna hole closure in mice taken on day 42 after injury after administration of 48 mg zebularine (Zeb) in 0.2 ml 2% alginate hydrogel and 0.8 mg retinoic acid (RA) dissolved in 20 µl of rapeseed oil, and then suspended in 0.2 ml of 2% alginate hydrogel (right panel) and a photo from the control experiment where the vehicle alone was administered (left panel).

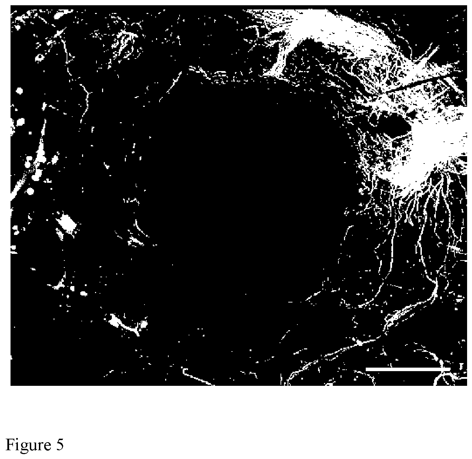

Figure 5 shows microscopic photo showing peripheral nerve growth in the area of regeneration in the auricle after administration of the alginate formulations of zebularine and retinoic acid on day 42 after injury. The presence of nerve fibres in the regenerated tissue is detected by staining with a neuron-specific fluorescently labelled anti-tubulin beta 3 antibody (*Tubb3*) (white in the figure).

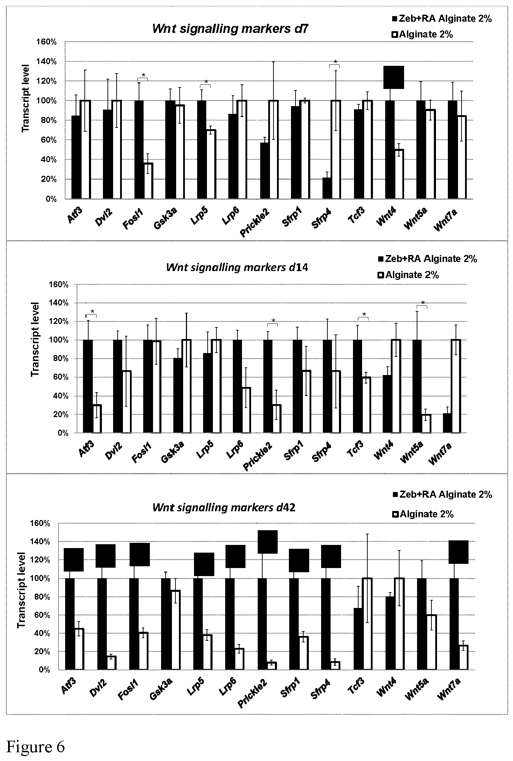

Figure 6 shows the expression changes of Wnt signalling genes observed in the regenerating ear pinnae in mice after subcutaneous administration of a 2% alginate hydrogel formulation with zebularine (Zeb) and retinoic acid (RA) and in the control mice receiving alginate hydrogel alone, on days 7, 14 and 28 post-injury. Mean gene expression levels were determined relative to the reference genes for 3 mice (n = 6 ears) for each time point. The statistical significance of the results was determined using the Mann-Whitney two-tailed test. Differences with statistical significance p <0.05 are marked with an asterisk (*), p <0.01 are marked with two asterisks (**). Error bars correspond to SEM (standard error mean).

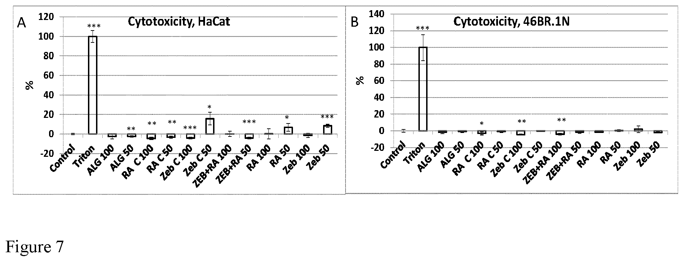

Figure 7. Cytotoxicity of alginate hydrogel formulations tested in cultured HaCaT keratinocytes (A) and 46BR.1N fibroblasts (B) using the LDH assay. The cultured cells were treated with extracts collected from alginate hydrogel formulations incubated with DMEM HG medium. Statistically significant differences were determined relative to the Control if not indicated otherwise using the heteroscedastic two-tailed Student's t-test and marked with single, double or triple asterisks as follows: * - p<0.05; ** - p<0.01, ^{∗∗∗} - p<0.001. Each experiment was performed in 4 replicates; error bars represent SD.
   Control - cells cultured in DMEM HG medium without serum; Triton - cells grown in DMEM HG medium with 1% (v/v) Triton X-100; ALG 100% - 200 µl of extract collected from 2% hydrogel alginate; ALG 50% 100 µl of extract collected from 2% hydrogel alginate + 100 µl of DMEM HG medium; ZEB C 100% - 100 µl of extract collected 2% hydrogel alginate with zebularine (48 mg per 1 ml ) + 100 µl of extract collected from 2% hydrogel alginate; ZEB C 50% - 50 µl of extract collected from 2% hydrogel alginate with zebularine (48 mg per 1 ml ) + 50 µl of extract collected from 2% hydrogel alginate + 100 µl of DMEM HG medium; RA C 100% - - 100 µl of extract collected 2% hydrogel alginate with retinoic acid (0.8 mg per 1 ml ) + 100 µl of extract collected from 2% hydrogel alginate; RA C 50% - 50 µl of extract collected from 2% hydrogel alginate with retinoic acid (0.8 mg per 1 ml ) + 50 µl of extract collected from 2% hydrogel alginate + 100 µl of DMEM HG medium; RA 100% - 200 µl of extract collected from 2% hydrogel alginate with retinoic acid (0.8 mg per 1 ml ); RA 50% - 100 µl of extract collected from 2% hydrogel alginate with retinoic acid (0.8 mg per 1 ml ) + 100 µl of DMEM HG medium; ZEB 100% - 200 µl of extract collected from 2% hydrogel alginate with zebularine (48 mg per 1 ml ); ZEB 50% - 100 µl of extract collected from 2% hydrogel alginate with zebularine (48 mg per 1 ml ) + 100 µl of DMEM HG medium; ZEB+RA 100% - 100 µl of extract collected from 2% hydrogel alginate with zebularine (48 mg per 1 ml ) + 100 µl of extract collected from 2% hydrogel alginate with retinoic acid (0.8 mg per 1 ml ); ZEB+RA 50% - 50 µl of extract collected from 2% hydrogel alginate with zebularine (48 mg per 1 ml ) + 50 µl of extract collected from 2% hydrogel alginate with retinoic acid (0.8 mg per 1 ml ) + 100 µl of DMEM HG
Figure 8. Effect of alginate hydrogel formulations on cell viability tested in cultured HaCaT keratinocytes (A) and 46BR.1N fibroblasts (B) using the XTT assay. The cultured cells were treated with extracts collected from alginate hydrogel formulations incubated with DMEM HG medium. Statistically significant differences were determined relative to the Control if not indicated otherwise using the heteroscedastic two-tailed Student's t-test and marked with single, double or triple asterisks as follows: * - p<0.05; ** - p<0.01, *** - p<0.001. Each experiment was performed in 4 replicates; error bars represent SD.
   Control - cells cultured in DMEM HG medium without serum; FBS cells grown in DMEM HG medium with 10% FBS; ALG 100% - 200 µl of extract collected from 2% hydrogel alginate; ALG 50% 100 µl of extract collected from 2% hydrogel alginate + 100 µl of DMEM HG medium; ZEB C 100% - 100 µl of extract collected 2% hydrogel alginate with zebularine (48 mg per 1 ml ) + 100 µl of extract collected from 2% hydrogel alginate; ZEB C 50% - 50 µl of extract collected from 2% hydrogel alginate with zebularine (48 mg per 1 ml ) + 50 µl of extract collected from 2% hydrogel alginate + 100 µl of DMEM HG medium; RA C 100% - - 100 µl of extract collected 2% hydrogel alginate with retinoic acid (0.8 mg per 1 ml ) + 100 µl of extract collected from 2% hydrogel alginate; RA C 50% - 50 µl of extract collected from 2% hydrogel alginate with retinoic acid (0.8 mg per 1 ml ) + 50 µl of extract collected from 2% hydrogel alginate + 100 µl of DMEM HG medium; RA 100% - 200 µl of extract collected from 2% hydrogel alginate with retinoic acid (0.8 mg per 1 ml ); RA 50% - 100 µl of extract collected from 2% hydrogel alginate with retinoic acid (0.8 mg per 1 ml ) + 100 µl of DMEM HG medium; ZEB 100% - 200 µl of extract collected from 2% hydrogel alginate with zebularine (48 mg per 1 ml ); ZEB 50% - 100 µl of extract collected from 2% hydrogel alginate with zebularine (48 mg per 1 ml ) + 100 µl of DMEM HG medium; ZEB+RA 100% - 100 µl of extract collected from 2% hydrogel alginate with zebularine (48 mg per 1 ml ) + 100 µl of extract collected from 2% hydrogel alginate with retinoic acid (0.8 mg per 1 ml ); ZEB+RA 50% - 50 µl of extract collected from 2% hydrogel alginate with zebularine (48 mg per 1 ml ) + 50 µl of extract collected from 2% hydrogel alginate with retinoic acid (0.8 mg per 1 ml ) + 100 µl of DMEM HG medium.

### Example 1

### Preparation and testing of alginate hydrogel formulations

Alginate hydrogel formulations were prepared as follows. First, a 2% sodium alginate solution was prepared by suspending 20 mg of pure sodium alginate in 1 ml of water. Zebularine formulations were prepared by suspending 240 mg of zebularine in 1 ml of 2% sodium alginate by 30 s homogenization in a bead mill homogenizer using 2 mm porcelain beads at a speed of 4 m/s at room temperature. The retinoic acid formulation was prepared in two ways: either by suspending 4 mg of retinoic acid in 1 ml of 2% sodium alginate or by suspending 4 mg of retinoic acid dissolved in 0.1 ml of rapeseed oil in 1 ml of 2% sodium alginate previously prepared. In both cases, the suspensions were homogenized in a bead mill using 2 mm porcelain spheres at a speed of 4 m/s at room temperature. The obtained formulations were examined using a light microscope, which showed the homogeneity of the zebularine alginate formulations and the alginate formulation of retinoic acid with the addition of rapeseed oil. The homogeneity of the zebularine hydrogel formulation was confirmed by microscopic observations that showed no crystals or agglomerates (Fig 1). The formulation of retinoic acid without the addition of rapeseed oil contained fine crystals of retinoic acid, invisible to the naked eye, with a uniform distribution in the hydrogel (Fig. 1). The oil formulation is in an emulsion-like form. The microscopic image of the hydrogel formulation of retinoic acid indicates that, although retinoic acid does not dissolve in the hydrogel, which is typical of hydrophobic compounds, fine crystals of retinoic acid are dispersed throughout the entire volume of the hydrogel. Thanks to this dispersion, retinoic acid will be gradually released into the system along with the biodegradation of alginate. All three formulations were injectable. The study performed in a Franz chamber showed that zebularine was gradually released from 2% alginate hydrogel (Fig. 2). The release of zebularine is evidenced by the increasing level of zebularine in time, expressed in arbitrary units (a.u.). For example, after 120 min the level of released zebularine was twice as high (4×10E11 a.u.) than after 1 h (2×10E11 a.u.). After 50 h (3000 min), the level of zebularine reached its maximum value of 1x10E12 a.u., more than after 30 min (1×10E11 a.u.). No release of retinoic acid from alginate formulations was observed under the Franz chamber conditions. As alginate undergoes biodegradation under the skin, thus allowing the gradual release of retinoic acid *in vivo,* the absence of retinoic acid release *in vitro* observed in a Franz chamber does not constitute a disadvantage.

### Example 2

Stimulating the regeneration of complex tissue after administering the composition, i.e. two hydrogel formulations of alginate with zebularine and with retinoic acid, the preparation of which is described in Example 1

The evaluation of the suitability of the alginate hydrogel formulations with zebularine and with retinoic acid, containing up to 240 mg of zebularine and up to 4 mg of retinoic acid per 1 ml of 2% alginate hydrogel to stimulate the process of complex tissue regeneration was carried out using a model of ear pinna punch wound in mice. Wounds were made with a laboratory scissor-style ear punch by cutting 2 mm diameter through-and-through holes in the middle part of ear pinnae. Female mice of the BALB/c strain, 8-10 weeks old on the starting day of the experiment, were used. The animals were randomized into two groups of six. Mice from the first group were administered subcutaneously with 48 mg of zebularine in 0.2 ml of 2% alginate hydrogel and 0.8 mg of retinoic acid in 0.2 ml of 2% alginate hydrogel as two consecutive injections immediately after wounding. The mice of the second group, the controls, received the subcutaneous injection of the vehicle alone (2% alginate hydrogel).

The protocol for animal experiments was approved by the Local Ethics Committee for Animal Experimentation in Bydgoszcz (permit no. 50/2016). The ears were photographed weekly for 6 weeks. The ear hole areas were determined by computer analysis of photographic documentation to assess regeneration effects. The mean percentages of ear hole closure on day 42 post-injury were 41.73 ± 8.47% SEM for the control mice and 74.13 ± 2.79 SEM% for the mice receiving the hydrogel formulations of zebularine and retinoic acid(p = 0.005). The progress of the regeneration process is shown in Figure 3. Statistically significant improvements in the ear hole closure in the mice treated with the hydrogel formulation of zebularine and retinoic acid were recorded from 7 to 42 days post-injury. Representative photographs of the mouse ears taken on days 42 after the injury demonstrate the regeneration of complex tissue upon the administration of an alginate hydrogel formulation with zebularine and retinoic acid (Figure 4); ear hole size reduction is the measure of the regenerative response. Extensive growth of small peripheral nerves within the reconstituted ear pinna tissue is observed, as shown by the signals from fluorescently labelled antibodies against nerve-specific tubulin beta 3 (Figure 5). Tissue innervation is essential because tissues lacking innervation are functionally deficient. The post-mortem dissections of the animals on day 42 showed no remains of alginate formulations under the skin, thus confirming biodegradation. The biodegradation of the hydrogel carrier explains the biological effect of retinoic acid in the animal's body, even though the release of this compound from alginate hydrogel was not observed *in vitro.*

### Example 3.

Effect of zebularine and retinoic acid administered in alginate hydrogel formulations on the expression of Wnt signalling genes in regenerating tissues

As the activation of Wnt signalling is typical of regeneration processes, gene expression levels of selected Wnt markers in the ear pinna wounds were compared between the mice receiving the alginate hydrogel formulations of zebularine and retinoic acid and the controls receiving alginate hydrogel alone on days 7, 14 and 42 post-injury. The panel of key Wnt signalling markers tested included 13 genes: *Atf3, Dvl2, Fosl1, Gsk3a, Lrp5, Lrp6, Prickle2, Sfrp1, Sfrp4, Tcf3, Wnt4, Wnt5a, Wnt7a.* For tissue collection, after the experiments as described in Example 2, the animals were euthanized on days 7, 14 and 42 after wounding and the rings surrounding the wounds were excised from the auricle using a 3 mm biopsy punch. The collected tissues were secured in the RNAlater reagent and stored at -80°C. After tissue disintegration with a hand-held mechanical homogenizer, RNA was extracted with the RNeasy kit (Qiagen). Transcript levels were determined by real-time quantitative PCR using *Tbp* and *Actb* as reference genes. The nucleotide sequences of the PCR primers are listed in Table 1.

Statistically significant increases in the expression of the Wnt genes after administration of zebularine and retinoic acid hydrogel formulations relative to the control mice were observed for *Fosl1, Lrp5* and *Wnt4* on day 7, for *Atf3, Prickle2, Tcf3* and *Wnt5a* on day 14, and for most of the tested genes, i.e. *Atf3, Dvl2, Fosl1, Lrp5, Lrp6, Prickle2, Sfrp1, Sfrp4, Wnt7a* on day 42 post-injury (Figure 6).

### Example 4 Cytotoxicity and the effect on cell viability of alginate hydrogel formulations with zebularine and retinoic acid

Cells were stimulated with the hydrogel extracts for the cytotoxicity and cell viability tests. The extracts were obtained by incubating 2% alginate hydrogel formulations with zebularine or retinoic acid or both and 2% alginate hydrogel alone in DMEM HG medium for 24 h at 37°C at a surface-to-volume ratio of 3cm2/ml. Then, the extracts were collected, centrifuged, and immediately used for cell stimulation. The cytotoxicity and cell viability were tested on cultured human HaCaT keratinocytes and 46BR.1N fibroblasts using the LDH and XTT assays, respectively. The cells were seeded into 96-well plates in DMEM HG medium containing 10% FBS. The cells were allowed to attach to a plate for 24 h in standard conditions (5% CO₂, 37°C). Next, the medium was removed, and cells were stimulated with the extracts. After 24-h incubation in standard conditions, half of the culture medium was collected for the cytotoxicity analysis with the LDH assay (Sigma Aldrich) and the other half for the analysis of cell viability performed with the XTT method (Sigma Aldrich). Both tests were conducted according to the manufacturer's instructions. The analysis was performed according to ISO10993 guidelines.

As demonstrated in Figure 7, the LDH assay did not show cytotoxicity of the tested extracts. The effects on cell viability are presented in Figure 8. While cell viability was not altered by alginate hydrogel alone, the extracts of zebularine alginate hydrogel formulations significantly decreased the viability of both the HaCat keratinocytes and 46BR.1N fibroblasts by approximately 30 and 40%, respectively. This impact was partially but statistically significantly neutralized by the addition of the extract obtained from alginate hydrogel alone. The extracts obtained from retinoic acid hydrogel formulations significantly decreased the viability of the keratinocyte (by approximately 20%) but not the fibroblast cells. This harmful retinoic acid extract's effect on cell viability was neutralized by the addition of the extract obtained from alginate hydrogel alone. The zebularine-mediated decrease in cell viability was partially but statistically significantly reversed by the retinoic acid extract.

Concluding, the changes in cell viability confirm the release of zebularine and retinoic acid from alginate hydrogel. The observed reduction in cell viability was significant but moderate, considering the high load of the active substances in the hydrogel formulations. The absence of cytotoxicity demonstrates a good safety profile of the alginate hydrogel formulations of zebularine and retinoic acid. The absence of cytotoxicity and adverse effects on cell viability after the treatment with the extracts obtained from pure alginate hydrogel confirmed the biocompatibility and safety of the carrier.

The examples confirm that:
After administration to the body, the mixture biodegrades, ensuring the gradual release of retinoic acid and zebularine, which enables stimulation of tissue regeneration; the therapeutic agent stimulates nerve growth, and the therapeutic agent activates the transcription of cell signalling genes essential for regeneration. Hydrogel biodegradation is described at the end of Example 2. Post-mortem sections determined no hydrogel remains under the skin of the animals. Alginate hydrogel shows no cytotoxicity and even partially neutralizes the negative effect of zebularine on cell viability, as shown in example 4.

**Table 1**

| Nucleotide sequences of the primers were used to test the levels of selected transcripts by real-time PCR. | | |
|---|---|---|
| Gene | Sense starter | Antisense starter |
| *Actb* | GGCTGTATTCCCCTCCATCG | CCAGTTGGTAACAATGCCATGT |
| *Tbp* | GAGAGCCACGGACAACTGCG | GGGAACTTCACATCACAGCTC |
| *Atf3* | TTTGCTAACCTGACACCCTTTG | AGAGGACATCCGATGGCAGA |
| *Dvl2* | GTAGGCGAGACGAAGGTGATT | CGCTGCAAAACGCTCTTGAA |
| *Fosl1* | ATGTACCGAGACTACGGGGAA | CTGCTGCTGTCGATGCTTG |
| *Gsk3a* | CAAGTTCCCCCAGATCAAAGC | GGCTAGAGCAGAGTGCAATGG |
| *LrpS* | ACGTCCCGTAAGGTTCTCTTC | GCCAGTAAATGTCGGAGTCTAC |
| *Lrp6* | TGCAAACAGACGGGACTTGAG | CGGGGACAATAATCCAGAAACAA |
| *Prickle2* | CATCAGCAAGCTCATGTTTGAC | ACCCAGGCGTACTCTTCCA |
| *Sfrp1* | TACTGGCCCGAGATGCTCAA | GAGGCTTCCGTGGTATTGGG |
| *Sftp4* | TCCATCCTGGTGGCGTTATG | GCATCCGGGTGATGTTCCA |
| *Tcf3* | TTTGACCCTAGCCGGACATAC | GCATAGGCATTCCGCTCAC |
| *Wnt4* | GTCAGGATGCTCGGACAACAT | CACGTCTTTACCTCGCAGGA |
| *Wnt5a* | AATGAAGCAGGCCGTAGGA | AGCCAGCACGTCTTGAGG |
| *Wnt7a* | TCAGTTTCAGTTCCGAAATGGC | CCCGACTCCCCACTTTGAG |

## Claims

1. Pharmaceutical composition comprising two hydrogel formulations with biologically active substances for simultaneous use, one mixture of which comprises not less than 60 mg zebularine added to 1 ml of sodium alginate hydrogel and not more than 240 mg of zebularine added to 1 ml of sodium alginate hydrogel, and the second mixture of which comprises not less than 1 mg of retinoic acid added to 1 ml of sodium alginate hydrogel and not more than 4 mg of retinoic acid added to 1 ml of sodium alginate hydrogel, wherein biologically active substances such as zebularine and retinoic acid release gradually from each formulation when administered to organism as a result of hydrogel biodegradation thus providing combined action and use for stimulating tissue regeneration.

2. Composition according to claim 1, wherein the sodium alginate is a 1 to 2% solution in water.
